# EUROPEAN PATENT SPECIFICATION

(11) **EP 2 609 217 B1**
(45) Date of publication and mention of the grant of the patent: **27.03.2019**
(21) Application number: 11760806.7
(22) Date of filing: 19.08.2011
(51) Int. Cl.: C12Q 1/6809, C12Q 1/6886

(54) **CELL CHARACTERISATION**
ZELLCHARAKTERISIERUNG
CARACTERISATION DE CELLULE

(30) Priority: 23.08.2010 GB 201014049
(43) Date of publication of application: 03.07.2013
(73) Proprietor: Sistemic Scotland Limited, Glasgow G20 0SP (GB)
(72) Inventor: O'BRIEN, Vincent, Ayr KA7 1JL (GB); HILLIER, Chris, Glasgow G74 4SN (GB)
(74) Representative: Ellis, Michael James
(86) International application number: PCT/GB2011/001241
(87) International publication number: WO 2012/025709

(56) References cited:
- WO-A1-2007/073737
- WO-A1-2008/064519
- WO-A1-2009/036332
- WO-A1-2009/062503
- WO-A2-2008/073915
- WO-A2-2008/112283
- WO-A2-2009/108866
- US-A1- 2010 003 674
- Ambion: "Variation of microRNA profiles in normal vs. cancerous hepatocytes", , 28 February 2009 (2009-02-28), XP002662921, Retrieved from the Internet: URL:http://www.ambion.com/techlib/tn/161/4 .html [retrieved on 2011-11-07]
- LANDGRAF P ET AL: "A Mammalian microRNA Expression Atlas Based on Small RNA Library Sequencing", CELL, CELL PRESS, US, vol. 129, no. 7, 29 June 2007 (2007-06-29) , pages 1401-1414, XP002490508, ISSN: 0092-8674, DOI: 10.1016/J.CELL.2007.04.040
- CALIN G A ET AL: "MicroRNA profiling reveals distinct signatures in B cell chronic lymphocytic leukemias", PROCEEDINGS OF THE NATIONAL ACADEMY OF SCIENCES OF USA, NATIONAL ACADEMY OF SCIENCE, WASHINGTON, DC; US, vol. 101, no. 32, 10 August 2004 (2004-08-10), pages 11755-11760, XP002339402, ISSN: 0027-8424, DOI: 10.1073/PNAS.0404432101
- MCCALLIE B ET AL: "Aberration of blastocyst microRNA expression is associated with human infertility", FERTILITY AND STERILITY, ELSEVIER SCIENCE INC, NEW YORK, NY, USA, vol. 93, no. 7, 1 May 2010 (2010-05-01), pages 2374-2382, XP027021097, ISSN: 0015-0282 [retrieved on 2009-03-17]
- Rajarshi Pal ET AL: "A panel of tests to standardize the characterization of human embryonic stem cells", REGENERATIVE MEDICINE, vol. 2, no. 2, 1 March 2007 (2007-03-01), pages 179-192, XP055247978, GB ISSN: 1746-0751, DOI: 10.2217/17460751.2.2.179
- CAIFU CHEN ET AL: "Defining embryonic stem cell identity using differentiation-related microRNAs and their potential targets", MAMMALIAN GENOME, SPRINGER-VERLAG, NE, vol. 18, no. 5, 3 July 2007 (2007-07-03), pages 316-327, XP019540280, ISSN: 1432-1777, DOI: 10.1007/S00335-007-9032-6
- REN JIAQIANG ET AL: "MicroRNA and gene expression patterns in the differentiation of human embryonic stem cells", JOURNAL OF TRANSLATIONAL MEDICINE, BIOMED CENTRAL, LONDON, GB, vol. 7, no. 1, 23 March 2009 (2009-03-23), page 20, XP021050755, ISSN: 1479-5876, DOI: 10.1186/1479-5876-7-20
- BLIN GUILLAUME ET AL: "A purified population of multipotent cardiovascular progenitors derived from primate pluripotent stem cells engrafts in postmyocardial infarcted nonhuman primates", JOURNAL OF CLINICAL INVESTIGATION, AMERICAN SOCIETY FOR CLINICAL INVESTIGATION, US, vol. 120, no. 4, 1 April 2010 (2010-04-01) , pages 1125-1139, XP002617207, ISSN: 0021-9738, DOI: 10.1172/JCI40120
- KOH T C ET AL: "Identification and expression analysis of miRNAs during batch culture of HEK-293 cells", JOURNAL OF BIOTECHNOLOGY, ELSEVIER SCIENCE PUBLISHERS, AMSTERDAM, NL, vol. 140, no. 3-4, 25 March 2009 (2009-03-25), pages 149-155, XP026073213, ISSN: 0168-1656, DOI: 10.1016/J.JBIOTEC.2009.01.021 [retrieved on 2009-02-07]
- WOLFGANG WAGNER ET AL: "Replicative Senescence of Mesenchymal Stem Cells: A Continuous and Organized Process", PL O S ONE, PUBLIC LIBRARY OF SCIENCE, US, vol. 3, no. 5, 21 May 2008 (2008-05-21), pages E2213-1, XP002686801, ISSN: 1932-6203, DOI: 10.1371/JOURNAL.PONE.0002213
- KANTARDJIEFF A ET AL: "Developing genomic platforms for Chinese hamster ovary cells", BIOTECHNOLOGY ADVANCES, ELSEVIER PUBLISHING, BARKING, GB, vol. 27, no. 6, 1 November 2009 (2009-11-01), pages 1028-1035, XP027205985, ISSN: 0734-9750 [retrieved on 2009-05-24]
- Kitchener D. Wilson ET AL: "MicroRNA Profiling of Human-Induced Pluripotent Stem Cells", Stem Cells and Development, vol. 18, no. 5, 1 June 2009 (2009-06-01), pages 749-757, XP055063091, ISSN: 1547-3287, DOI: 10.1089/scd.2008.0247

## Description

### FIELD OF THE INVENTION

The present invention relates to uses of non-coding RNA in methods for characterising and/or profiling cells. In particular, the uses and methods described herein may be exploited to assess the quality, identity, purity, potency and safety of cells and/or cell cultures.

### BACKGROUND OF THE INVENTION

There has been rapid progress in biotechnology and medicine that has led to the development of new treatments and medicinal products, among them products containing viable cells. These new cell-based products have great potential in the treatment of various diseases where there is an unmet medical need. The cell products are, in the case of stem cells, used directly for therapeutic purposes or are research tools to aid drug discovery by providing a homogenous source of stem cells, cells committed to differentiate to one or more lineages or terminally-differentiated cells of a particular lineage. Mammalian cell lines used in research are vital tools for understanding basic biological concepts while cells used in bioprocessing applications can yield macromolecules used for research purposes or clinical applications.

### Current characterisation and safety testing methods.

There are a number of methods used to assess the quality, consistency and potency of stem cells and cell cultures. For stem cells this is defined as their self-renewal capacity and by the expression of specific markers. The identity of the desired cell population must be defined. Currently hESC lines are characterised using a set of standardised metrics: surface antigens, expression of particular enzymic activities (e.g. Alkaline phosphatase), gene expression, epigenetic markers, assessing genomic stability, cytology and morphology as well *in vitro* (embryonic body formation) and *in vivo* differentiation potential (formation of teratoma-like xenografts) and by the absence of measurable microbiological infections. However, the procedures used to assess these stem cell characteristics require skilled staff, but have a relatively low-information-content and are time-consuming and expensive. In addition they do not reveal crucial information on the safety profile and/or fitness-for-purpose of the resultant cells. There is a need for low-skill, low-cost, information-rich QC assays and kits that inform on the quality and consistency of the stem cell lines at derivation and under continued passage in culture, including, for stem cells, expansion of cell populations under conditions supporting proliferation of undifferentiated cells. These QC checks should also provide relevant biological information on their likely suitability for purpose and, if developed for clinical use, their safety for deployment.

There is a requirement to continuously assess the inherent heterogeneity of human-based cell products in order to seek to minimise this variation during the manufacturing of cell-based starting material. Correspondingly, there is a need for a relatively straightforward assay that reports on both phenotypic drift of cells in culture and provides an assessment of the likelihood of their safety profile (e.g. tumourigenicity) if the cells are used as medicinal products.

MicroRNAs (miRNAs) are single-stranded RNA molecules having a length of around 18 to 25 nucleotides. miRNAs were first described by Victor Ambros in 1993 and since then over 2,000 papers on have been published on the subject of miRNAs. There are predicted to be about 1,000 miRNAs in humans, although some estimates place the figure at tens of thousands. miRNA is not translated into protein but instead regulates the expression of one or more genes. Known biology currently shows that microRNAs target particular individual messenger RNAs (mRNAs) or groups of mRNAs, thereby preventing their translation or accelerating mRNA degradation. The mature single stranded miRNA molecule complexes with the RNA-Induced Silencing Complex (RISC) protein and binds to a partially complementary sequence within the 3'untranslated region (3'-UTR) of the protein coding mRNA from its target gene.

Further proteins are recruited to form a silencing complex and the expression of the target gene product is repressed by a mechanism that blocks the translation of the mRNA.

Although much remains to be discovered about the biology of miRNAs and the composition and mechanism of action of the silencing complex it is apparent that miRNAs are involved in the regulation of many genes. MiRNAs are thought to regulate as many as 30% of all genes (Xie et al, 2005) at the translational level. An miRNA can regulate multiple genes and each gene can be regulated by multiple miRNAs permitting complex interrelationships between miRNA/mRNA networks within tissues and cells.

Tissue-specific expression of miRNAs is thought to guide commitment of cells to differentiate and/or actively maintain cell or tissue identity. This wide-ranging influence and interplay between different miRNAs suggests that deregulated expression of a single miRNA or small sub-set of miRNAs may result in striking physiological or pathophysiological changes and complex disease traits (Lim et al, 2005). More than 50% of known human miRNAs reside in genomic regions prone to alteration in cancer cells (Calin et al, 2004). Not surprisingly, the expression pattern of miRNAs change in cancer and other disease states. This information has begun to be used to classify and stage cancers, reveal biomarkers for prognosis and response and provide a critical determinant to guide therapeutic intervention.

An increasing body of evidence confirms that the expression levels of individual miRNAs vary significantly between cell types or within a cell type maintained under different physiological conditions and so can be used to define the cell type, the physiological status of the cell and monitor response to environmental changes.

Embryonic and induced pluripotent stem cells are characterised by their ability to self- renew and differentiate into all cell types. The molecular mechanisms behind this process are complex and rely on the interplay between a network of transcription factors, epigenetic regulators, including miRNAs, and signalling pathways. MicroRNAs play essential roles in maintenance of pluripotency, proliferation and differentiation. Recent studies have begun to clarify the specific role of miRNA in regulatory circuitries that control self- renewal and pluripotency of both embryonic stem cells and induced pluripotent stem cells. These advances point to a critical role for miRNAs in the process of reprogramming somatic cells to pluripotent cells.

Pal et al. analysed protein and micro-RNA expression in a human embryonic stem cell line and suggested using their expression profiles in assays for determining their quality (Regenerative Med. (2007) 2(2), 179-192).

We have used the 'fingerprint' patterns extracted from the information content held within the miRNA expression profile of cells to monitor the maintenance of cell identity and functional capability. The miRNA profile provides a unique insight into cell biology and can be reduced to practice through the development of kits to monitor pluripotency, cell-fate, cell-identity and phenotypic drift over multiple passages using a single development platform for microRNA screening.

The invention aims to provide alternative methods for monitoring the quality and suitability of cells for the purpose for which they were developed.

### SUMMARY OF THE INVENTION

There is provided in a first aspect of the invention a method of quality assessing stem cells or induced pluripotent stem cells or their intermediate stages of differentiating to one or more terminal differentiating states for use in cell therapy for levels of cell contamination, said method comprising the steps of:
(i) determining a micro-RNA expression profile of the stem cells or induced pluripotent stem cells or their intermediate stages of differentiating to one or more terminal differentiating states to be assessed for a particular group or subset of micro-RNAs;
(ii) providing a reference micro-RNA expression profile of the particular group or subset of micro-RNAs which is derived from a cell sample of stem cells or induced pluripotent stem cells or their intermediate stages of differentiating to one or more terminal differentiating states that conforms to a predetermined standard for the use of stem cells or induced pluripotent stem cells or their intermediate stages of differentiating to one or more terminal differentiating states in the cell therapy;
(iii) comparing the micro-RNA expression profile determined in step (i) with the reference micro-RNA expression profile provided in step (ii); and
(iv) determining from the comparison of micro-RNA expression profiles a quality assessment as to the suitability of the stem cells or induced pluripotent stem cells or their intermediate stages of differentiating to one or more terminal differentiating states for use in the cell therapy.

In a second aspect of the invention, there is provided use of micro-RNA molecules for quality assessing stem cells or induced pluripotent stem cells or their intermediate stages of differentiating to one or more terminal differentiating states for use in cell therapy, wherein the qualities being assessed comprise levels of cell contamination.

### DETAILED DESCRIPTION

The disclosure concerns methods employing non-coding RNA expression assays as a means to characterise cells and/or to monitor the quality and safety profile of in vitro cell culture systems.

Aspects of the disclosure include, but are not limited to, determining the microRNA profile of cells and serial passages of an in vitro cell culture system. The term "cell" should be understood to encompass any eukaryotic cell. For example a "cell" within the context of this disclosure may be a mammalian (adult, foetal or embryonic) cell, including, for example a stem cell or iPS cell. The invention concerns stem cells or induced pluripotent stem cells (iPS) or their intermediate stages differentiating to one or more terminal differentiation states. The cells may be adult stem cells (tissue- specific progenitor cells or mesenchymal/stromal cells) or their intermediates differentiating to one or more terminal differentiation states under the influence of external factors in the culture medium.

The method reveals sample clustering based on their microRNA expression profile and identifies statistically valid, candidate microRNAs which are consistent and reliable markers of undesirable or uncharacterised alterations in the cell system being monitored and therefore provide key decision-support tools on the continued usefulness of the cell system for their intended therapeutic application.

The present disclosure concerns the finding that non-coding RNA profiles can be exploited as a means of monitoring, assessing, comparing, establishing and/or determining certain cell characteristics and/or profiles. In one instance, the various uses and/or methods described herein may be exploited to determine, monitor, establish, compare and/or assess cell characteristics which are also markers of cell quality and/or safety.

The inventors have determined that profiles of micro-RNA molecule expression (referred to hereinafter as micro-RNA expression profiles) provide a "fingerprint" which can be correlated to, linked or matched with, the presence of particular cell characteristics and/or certain cell profiles. By establishing a micro-RNA expression profile indicative of one or more cell characteristic(s) or a particular cell profile, it is possible to assess other cells for corresponding characteristics and/or profiles by simple comparison of the micro-RNA expression profiles. Additionally, the inventors have surprisingly discovered that cells which are shown to be phenotypically identical by standard analytical techniques (such as, for example by flow cytometry and/or cell surface/cytoplasmic/nuclear marker analysis and the like) can be shown by the micro-RNA profiling techniques described herein, to be genotypically (and thus most likely phenotypically) distinct/different. Where cell safety and quality are concerned, the phenotypic differences between an un-safe (for example tumorogenic) cell or cells and/or a cell of poor quality (perhaps lacking expression of specific markers), may be undetectable by standard techniques. The instant invention provides a highly sensitive an accurate means of establishing whether or not a cell or cell system (for example the population of cells within a cell culture) conforms to a set of predetermined standards. One of skill will appreciate that provided one establishes a micro- RNA profile of a cell which is known to conform to a set of predetermined safety and/or quality standards, other cells of the same type can be assessed for conformity with the predetermined safety and/or quality standards by comparison of micro-RNA.

In view of the above, one embodiment of this invention, further provides the use of micro-RNA molecules for characterising and/or profiling cells, wherein the cells are shown to be phenotypically identical to a reference cell by methods other than micro- RNA profiling. In one embodiment, the method by which the cell and a reference cell are shown to be identical may be flow cytometry. In this context, a reference cell may be a cell conforming to a predetermined set of safety and/or quality standards.

In one embodiment, the methods provided by this invention may exclude methods which exploit micro-RNA profiling to distinguish one differentiative cell state from another. For example, in some embodiments, the invention may not embrace the use of micro-RNA profiling to assess the differentiation of stem cells to other cell types.

Optionally, the qualities being assessed further comprise the maintenance of certain cell characteristics, such as cell identity, phenotypic stability and functional capability.

It should be understood that a cell "characteristic" or "profile" may relate to cell features such as identity (type), morphology, genotype, phenotype (to monitor phenotypic stability), viability, potency (for example degree of pluripotency), contaminant levels, safety (for example tumourigenicity) and/or quality. In certain embodiments, a cell "profile" may be determined by establishing aspects of one or more of a cell's morphology, genotype, phenotype, viability, potency (pluripotency), contaminant levels, safety (tumourigencity) and/or quality. One of skill will appreciate that the terms cell "characteristic" and/or "profile" may relate to the biological activity and/or compound secretion/production profile. By way of example, a cell characteristic and/or profile may relate to the ability of a cell to express, produce and/or secrete a natural of heterologous compound or compounds such as, for example, a protein, peptide, amino acid, nucleic acid, carbohydrate and/or other small organic compound.

The term "non-coding RNA" may include microRNA (miRNA) molecules and either or both miRNA precursors and mature miRNAs. The term may further include small interfering RNAs (siRNA), piwi-interacting RNAs (pi RNA), small nuclear RNA (snRNA) and short hairpin RNA (shRNA). "Non-coding RNA" may further comprise transgenic non-coding RNAs which may function as reporters of non- coding RNA expression. The non-coding RNAs may be episomal and the methods and/or uses described herein may require initial steps in which episomal DNA is introduced into the cells described herein whereupon the episomal DNA can be transcribed to produce non-coding RNA which constitutes all or part of the profiled non-coding RNA. In one instance, the term "non-coding RNA" does not include non-coding RNAs known as "teloRNA" or "teloRNA mark".

A micro-RNA expression profile may relate to the expression and/or identity of at least one micro-RNA. In one embodiment, the micro-RNA expression profile relates to the expression of a plurality of micro-RNAs. Accordingly, a micro-RNA expression profile may comprise some indication of the identity of one or more micro-RNAs expressed by a cell optionally together with quantitative and/or qualitative measurements of the level of expression of one or more micro-RNAs within a cell.

In certain embodiments, the methods and uses described herein may require the use of a micro-RNA expression profile database. Such a database may be referred to as a micro-RNA reference library. Micro-RNA databases described herein may comprise one or more reference micro-RNA profiles each being derived from a cell having known characteristics/profiles and/or cells which have been cultured according to a particular protocol and/or subjected to known or defined interventions.

In one embodiment, the reference micro-RNA profiles may be derived from an isolated cell, cells derived from a cell culture, cell line and/or stored cell preparation. Additionally or alternatively, the reference micro-RNA profiles may be obtained from cells subjected to one or more defined or predetermined interventions and/or cells subjected to a particular culture protocol, altered culture conditions and/or one or more interventions. The reference micro-RNA profiles described herein, may comprise micro-RNA profiles derived from single cell types and/or a plurality of different cell types. In other embodiments, the reference micro-RNA profile may be derived from primary cell cultures and/or immortalised cells. Advantageously, the reference micro-RNA profile is obtained from a cell or cell exhibiting known and/or desired characteristic(s), a desired and/or correct profile and/or an cell or cells which meet a certain predetermined quality and/or standard.

Since the reference micro-RNA expression profiles are derived from cells exhibiting known (desirable) characteristics and/or profiles, one of skill will appreciate that any cell which exhibits a comparable micro-RNA profile, must possess similar characteristic(s) or a similar profile.

The reference micro-RNA expression profiles may be compiled using multiple sets of data obtained from repeat micro-RNA expression analysis of cells having known characteristics and/or known profiles and/or from micro-RNA expression analysis of cells conforming to known or approved standards.

For convenience, the reference micro-RNA profiles described herein may be referred to as "comparative micro-RNA profiles".

The process of comparing micro-RNA expression profiles obtained from cells to be quality assessed, with reference micro-RNA profiles (optionally contained within a database) as described herein, may involve identifying correlations between micro-RNA profiles. Correlations between micro-RNA profiles of cells being quality assessed are typically correlations, positive or negative, between changes in the expression of one or more micro0RNAs. For example, a positive correlation may comprise the identification of a particular micro-RNA profile in a cell being quality controlled and the same non-coding RNA profile in reference micro-RNA profile (or database). A negative correlation may comprise the identification of a particular micro-RNA profile in a cell being quality controlled and a reference micro-RNA profile which, while exhibiting expression of corresponding micro-RNAs exhibits variable or differential expression levels (i.e. the expression of a particular micro-RNA in a reference profile may be less than when compared to the expression of the same micro-RNA identified in a cell being quality controlled).

The reference profiles and/or databases described herein may comprise micro-RNA expression profiles which have been categorised (clustered or grouped) on the basis of similarities present in the reference micro-RNA profiles. For example, data relating to particular cell types and/or to cells cultured in a particular way, may be grouped together so as to facilitate probing a database for correlations with micro-RNA profiles of cells being characterised, profiled and/or quality controlled.

In view of the above, the micro-RNA profiles contained within the reference profiles may represent the profiles of one or more types of cell, cells at various stages of culture, cells cultured according to particular protocols and/or cells subject to one or more interventions 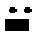 perhaps an interventionoccurring during culture.

The term "intervention" may be taken to include the act of administering a compound or compounds to a cell. In other embodiments, an intervention may include the change of culture media, the addition of one or more media supplements as well as alterations in culture conditions such as, for example, time, temperature, pH and/or osmolarity. An intervention may also include the transfer of cells from one culture vessel to another 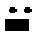 perhaps as a result of cell sub-culturing procedures.

The present methods find particular application in the field of cell culture where it may be necessary to ensure that one or more cell interventions or protocols has not had a deleterious effect on the cells of the cell culture. For example, by compiling a reference profile of cells which exhibit favourable or desired characteristics before during and/or after successful culture according to one or more protocols, it may be possible to establish whether other cells cultured according to the same protocols exhibit the same characteristics before, during and/or after culture, by simple comparison of micro-RNA profiles.

Where the reference micro-RNA profiles are intended to represent the characteristics and/or features of cells being cultured, micro-RNA profiles may be obtained from serially passaged (split and/or subcultured) cultures of cells either at or during each passage and/or at various other points during culture. Additionally, or alternatively, when culture conditions are altered or the cells of the culture are subject to an intervention (perhaps the addition of a supplement (antibiotic, nutrient or the like), a reference micro-RNA expression profile may be obtained.

In this way, it is possible to construct a database comprising one or more reference micro-RNA profiles which reflect the micro-RNA profiles of cells in culture. One of skill will appreciate that such a database may be used to monitor and/or assess cell cultures by comparison of the micro-RNA profiles of cells from the cell culture with the reference micro-RNA profiles of the database.

In one instance, the methods provided may further be used to assess the effect of specific culture substrates (or components thereof) on cells and cell cultures. For example, the methods may be further exploited as a means of assessing or monitoring the performance of nanofibres/nanoscale growth surfaces which can be used to maintain the pluripotency or a specific differentiative state of stem cells. In such cases, a micro-RNA profile indicative of a pluripotent cell or correctly differentiated cell would be obtained and compared to the micro-RNA profile of cell cultured on a nanofibres/nanoscale growth surface in order to determine whether or not the cells remain pluripotent or correctly differentiated.

In other instance, the micro-RNA profiling methods provided may be further exploited to assess the effectiveness of a lyophilisation technique or the viability of cells subjected to such a process. Again, comparative micro-RNA profiles would be obtained from cells before and after a lyophilisation process and/or cells which remain viable after lyophilisation. Such techniques could be applied to erythrocyte lyophilisation protocols.

In yet further instances the micro-RNA profiling provided may further be used to assess the effectiveness of protocols which force the differentiation of one cell type from another. Such protocols may include those which cause differentiation without a pluripotent intermediate. By way of example, the micro-RNA profiling methods may be further used to assess the success of a fibroblast/erythrocyte differentiation protocol, a comparative micro-RNA profile being obtained from a correctly differentiated erythrocyte cell.

Micro-RNA expression profiles may be measured or determined for each micro-RNA within a particular group or subset of micro-RNAs. Additionally, or alternatively, micro-RNA expression profiles may comprise the identification of an individual micro-RNA and measuring and/or determining the expression thereof. The level of expression may be determined indirectly via measurements of the amount or level of activation of a reporter construct, for example a transgenic reporter construct incorporated into the genome of a cell.

The methods and uses of this invention find particular application in cell quality control and/or safety analysis procedures. One of skill in this field will appreciate that commercial production, sale and distribution of cells particularly cells derived from stored cell lines, is subject to stringent quality and safety control, primarily to ensure that stored cells and/or cells distributed to customers, meet certain predetermined standards. For example it may be necessary to ensure that cells cultured from stored cell lines are as described (both in terms of identity and morphology), are viable and exhibit certain characteristics (features and/or traits).

Current cell quality control processes or procedures, may involve a series of complex, time consuming and costly tests each of which is designed to confirm that a cell meets a pre-determined standard. Such tests may be performed prior to shipping a cell line to a customer but also at regular intervals during storage or culture. By way of example, cell quality control procedures may comprise tests designed to assess cell identity/morphology, cell phenotype, cell genotype, levels of cell contamination, degree of pluripotency, cell viability and/or cell safety. Such tests may involve the use of DNA profiling techniques, immunohistochemistry, alkaline phosphatase staining, flow cytometry, gene expression analysis (perhaps using expression arrays and the like), blood group typing, karyology, microorganism screening (using PCR and immunological based techniques), teratoma and embryoid body formation (particularly relevant where the pluripotency of a stem cell is being tested) and simple live/dead (trypan blue) stains to determine viability.

By establishing a reference or comparative micro-RNA profile indicative of a certain cell "standard" or "quality standard", it is possible to quality control cells by comparison of micro-RNA profiles. By way of example, the micro-RNA profile of a cell cultured from a stored cell line may be compared with the micro-RNA profile (i.e. a reference micro-RNA profile) of the same type of cell which is known to meet one or more predetermined standards. If the micro-RNA profile of the cell being cultured is comparable to, or matches with, the (reference) micro-RNA profile derived from a cell known to meet one or more pre-determined standards, one may conclude that the cultured cell meets the same standards.

It should be understood that the term "standard" or "quality standard" may relate to defined criteria or features which any given cell must exhibit prior to being used (in anyway whatsoever), sold or distributed. Such standards may be set by regulatory bodies but may also relate to locally determined cell features and/or characteristics which render cells suitable for particular uses for example uses in assays and the like.

In view of the above, the present invention provides use of micro-RNA profiles in cell quality control, wherein micro-RNA molecules are used for quality assessing stem cells or induced pluripotent stem cells or their intermediate stages of differentiating to one or more terminal differentiating states for use in cell therapy, wherein the qualities being assessed comprise levels of cell contamination.

In a further instance, the disclosure provides a method of quality controlling cells, comprising the steps of comparing the micro-RNA profile of cells to be quality controlled, with a reference micro-RNA profile. In one instance, the reference micro-RNA profiles may be derived from a cell or cells known to meet a certain quality standard. Since the reference micro-RNA profiles are derived from a cell meeting one or more predetermined standard(s), any cell which exhibits a micro-RNA profile corresponding to a reference micro-RNA profile, must be of a similar quality standard. In one instance, the micro-RNA profile of the cell to be quality controlled may be compared with a database comprising one or more reference micro-RNA profiles.

In one embodiment, the quality control procedures further comprise establishing the identity, phenotype, genotype, viability and/or pluripotency in stored and/or cultured cells.

Advantageously, the reference micro-RNA profiles described herein may be derived from cells of known identify and having defined phenotypes and/or genotypes, known levels of contamination (low/no contamination, moderate or high levels of contamination), defined pluripotency (for example complete, partial or no pluripotency), and defined levels of viability.

For example, methods for assessing the pluripotency of a cell may comprise the step of comparing the micro-RNA profile of a cell with unknown pluripotency with the micro-RNA profile of the same type of cell having a known level of pluripotency.

Similarly, cell identity may be confirmed by comparing the micro-RNA profile of a cell (perhaps a cell of unknown identity) with the micro-RNA profiles of a cell of known identity. If the micro-RNA profile of the unknown cell corresponds to, or matches with, the micro-RNA profile of any of the known cells, then it may be concluded that the unknown cell is the same as the cell from which the corresponding or matching micro-RNA profile was derived.

In one embodiment, the methods described herein may be exploited to establish a level of Mycoplasma contamination in a cell or cells. One of skill will appreciate that a comparative or reference micro-RNA profile may be obtained from a corresponding cell type or cell population known to be free from Mycoplasma contamination.

One of skill will appreciate that the present invention, and in particular those embodiments relating to cell quality control, finds particular application in the field of cell culture, particularly commercial cell culture where large numbers of cells are stored and cultured.

When culturing cells, it is often important to make regular checks to ensure that the cultures comprise cells which meet certain predetermined standards. For example, beyond establishing that the cultured cells are of the correct cell type, it may be necessary to ensure that the cell expresses certain markers or that the cell expresses a particular compound or compounds or that interventions which occur during cell culture do not have a deleterious effect upon the cells. Where the cell culture comprises stem cells, it may be necessary to ensure that the cells of the culture comprises cells which remain pluripotent throughout passage and/or that the cell follows a particular differentiation path. By comparing the micro-RNA profiles of cultured cells with the micro-RNA profiles of cultured cells conforming to known or predetermined culture standards, it is possible to ensure that the cells being cultured meet those same standards.

In one instance, a database comprising one or more reference micro-RNA profiles may comprise micro-RNA profiles obtained from cells being serially passaged and at various stages of culture. For example, the database may comprise the micro-RNA profiles of one or more different types of cells during early-, mid- and/or late-phase passage or culture or at any other time point there between. Additionally or alternatively, the database may contain the micro-RNA profiles of cells which have been subjected to some form of altered culture condition (for example altered time, temperature, pH, nutrient and/or metabolite availability). In another instance, the database may contain micro-RNA profiles obtained from one or more cells which have been contacted with various agents such as, for example, growth media supplements including, vitamins, nutrients, nucleic acids, antibiotics, candidate drug compounds, test agents, antibodies, carbohydrates, proteins, peptides and/or amino acids. It should be understood that the database may contain many such profiles obtained from a variety of different cell types.

One of skill will appreciate that the data comprising the reference micro-RNA profiles may be compared with data from cells being tested, with the aid of data processing/analysis techniques such as, for example statistical mathematical methods. For example, techniques such as principle component analysis or pattern recognition algorithms may be used to identify correlations between data contained within the database and micro-RNA expression profiles obtained from cells being tested.

The invention may be carried out by use of a kit for characterising, profiling and/or quality controlling cells, said kit comprising a database of one or more reference microRNA profiles and assay systems, apparatus and/or reagents necessary to obtain micro-RNA profiles from cells to be characterised, profiled and/or quality controlled. The user may simply obtain the micro-RNA profile of a cell to be characterised, profiled and/or quality controlled and simply compare the micro-RNA profile with the micro-RNA profile(s) of the database.

The present invention may find application in a quality control service whereby a service provider receives cells from third parties to be quality controlled. The service provider may have one or more micro-RNA databases of the type described herein and which can be used to compare the micro-RNA profiles of the cells provided by the third parties. Once the micro-RNA profiles of the cells provided by the third parties have been compared with the micro-RNA profiles of database, the third party may then be provide with a report detailing information relating to the quality of the cells.

Such a service may be particularly useful to third parties involved in cell storage and/or culture. The service may be of particular use to those who are required to make regular checks of cells in storage or culture to determine levels of contamination. Furthermore, the services described herein may be used to ensure that cells subjected to particular interventions or culture protocols possess the required characteristics before, during and after execution of the protocol and/or intervention.

The third party may further provide information relating to the culture protocols used to culture the cells and/or information relating to certain features, traits and/or characteristics the cells to be quality controlled, should have.

The following is a description in detail with reference to the following figures which show:
Figure 1 is a flow diagram of a method;

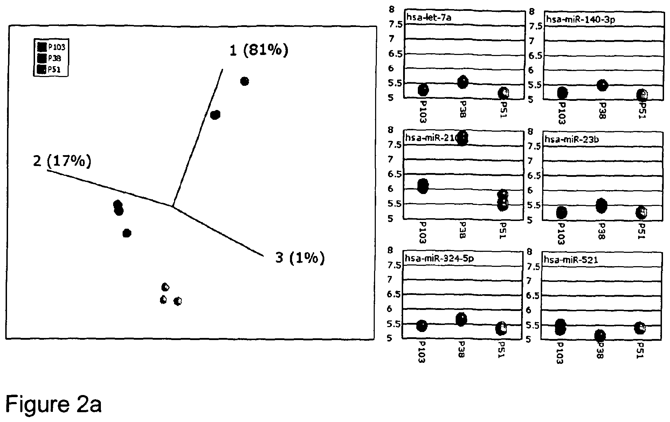

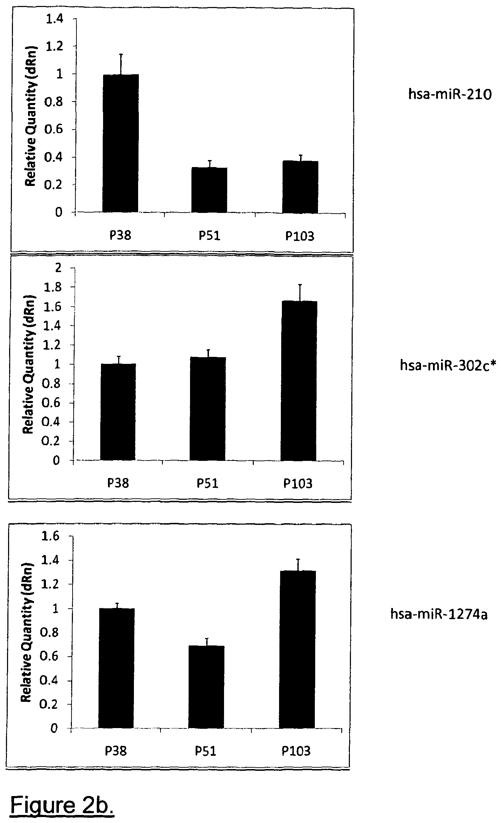

Figure 2 Decreased expression of hsa-miRNA-210 and increased expression of hsa-miR-1274a and hsa-miR-302c* with extend in vitro passage of hESCs with both microarray and QPCR data panels. Figure 2a: Left panel: Principal Components Analysis reveals separation of samples based on cell passage number in human embroyonic stem cell line RCM1. Right panel: expression profile analysis of microRNA microarray expression data (normalised signal intensities from the array) for hsa-miR-210 and three other microRNA which do not significantly change expression between passages. Figure 2b: Confirmation of key microRNA expression differences by qRT-PCR data

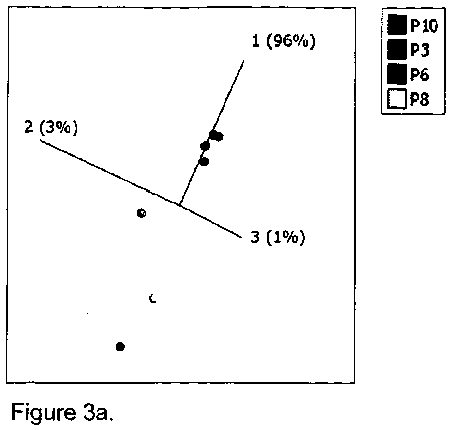

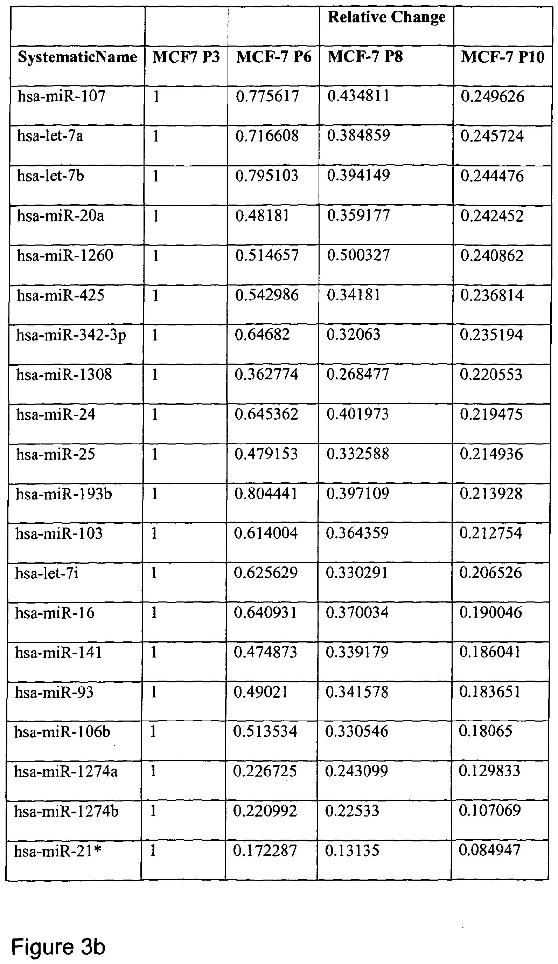

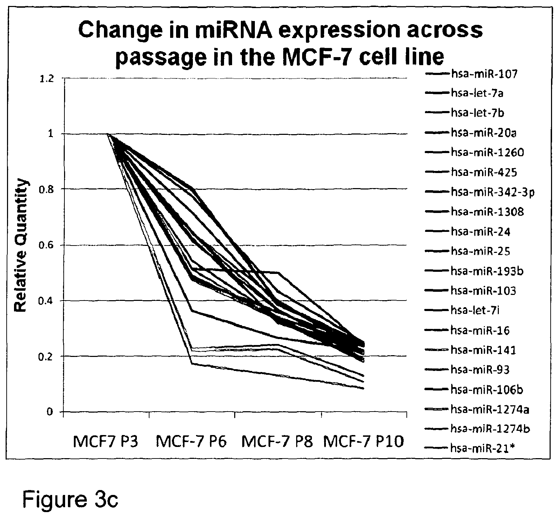

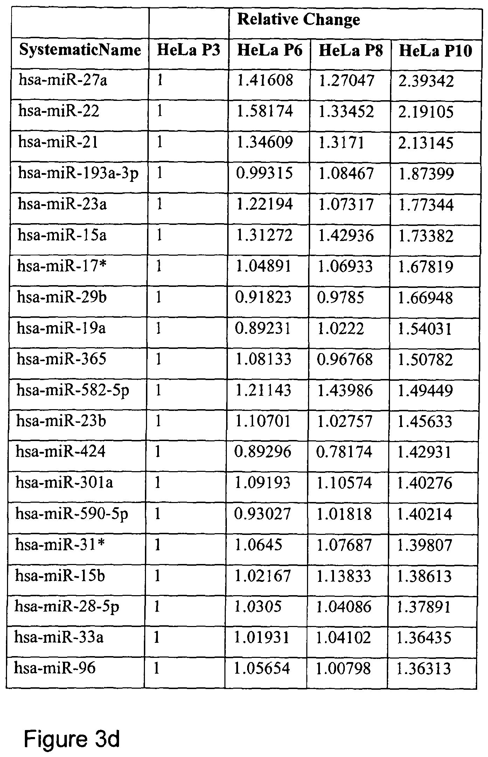

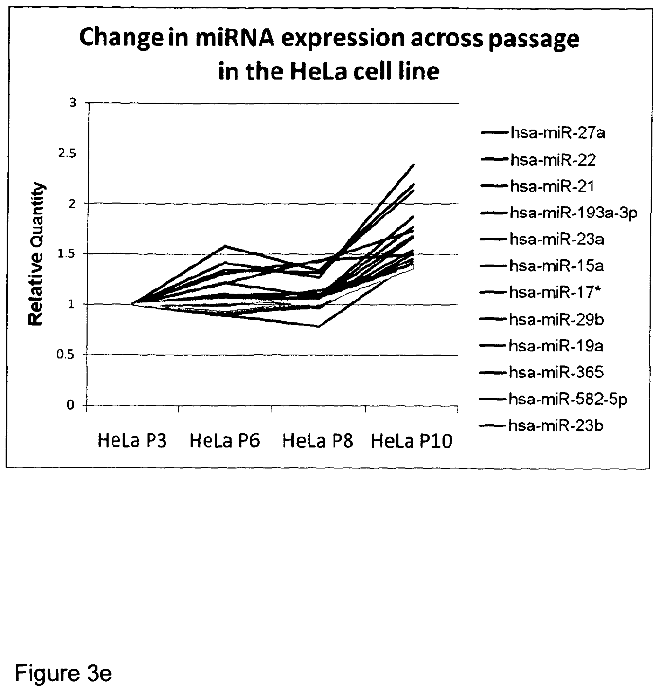

Figure 3. Phenotypic 'drift' of human cancer-derived cell lines (HeLa and MCF-7) with extended passaging in vitro. Figure 3a: Alterations in microRNA profiles in a serially passaged human, tumour-derived cell lines (HeLa and MCF-7); principal components analysis of microRNA datasets reveals separation of samples based on cell passage number in MCF-7 cells. Figure 3b and profile analysis (Figure 3c) below show twenty miRNAs altered during serial passage of MCF-7 cells in culture. All twenty miRNAs show significant decreases in gene expression over the seven passages monitored. The changes are shown as relative changes (fold changes) in comparison to the earliest passage (P3) cells. Figure 3d and profile analysis (Figure 3e) below show twenty miRNAs altereds during serial passage of HeLa cells in culture. All twenty miRNAs show significant alterations in miRNA expression over the seven passages monitored. The changes are shown as relative changes (fold changes) in comparison to the earliest passage (P3) cells.
Figure 4a. Flow Cytometry results for 2 hESC populations that are maintained under identical culture conditions for extended passages.
Figure 4b. Principal component analysis (PCA) of miRNA profile of the Mid- and High-passage hESC populations.
Figure 4c. A volcano plot representing the differential expression of microRNA between mid-passage (P51) and high-passage (PI03) cells. The 5 differentially-expressed miRNAs with a fold-change difference of 2 or more are circled in red.
Figure 4d. The identification of 5 microRNAs (circled red in figure 4) which demonstrate a greater than 2-fold differential expression between P51 and P103 hESC cultures.
Figure 5. Visualisation reveals clustering of different sample groups based on differences in miRNA expression profiles. **A.** Visualisation using principal component analysis (PCA) where the arrows denote the trajectories of differentiation **B.** Visualisation of sample relationships using hierarchical clustering and a heatmap.

### EXAMPLE 1

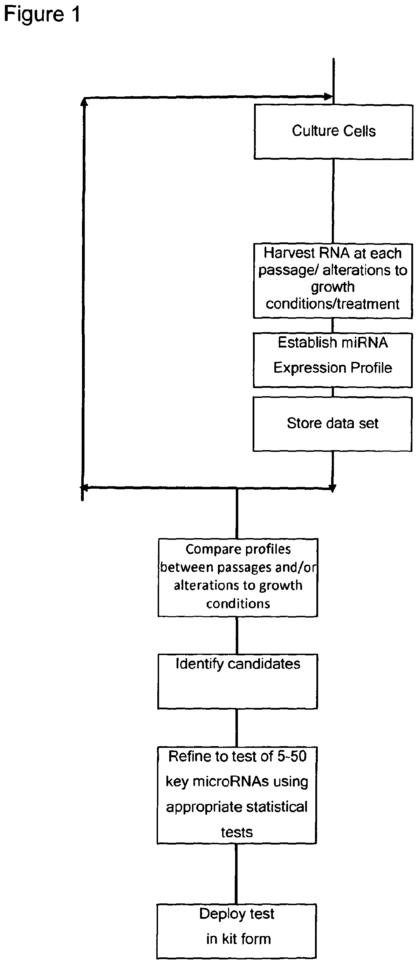

In an example application a database of miRNA expression data sets (being an example of an expression data set derived from a measured non-coding RNA expression profile) are prepared. With reference to Figure 1, suitable human embryonic stem cells are cultured by known methods over an extended period of time and sampled at 3 points after their derivation i.e. at passages 38, 51 and 103. A miRNA expression profile is then measured using a sample of the cells at each passage to determine the expression level of each of a number of miRNAs in the treated cells.

Two alternative methods for measuring the miRNA expression profiles, microarray analysis and qualitative real-time PCR analysis, are set out below.

### (1) miRNA microarray and data analysis

Total RNA from reference cells (n = 3) is isolated using a column-based kit from Exiqon A/S of Vedbaek, Denmark. Two µg of total RNA from each sample is analysed by miRNA microarray. miRNA microarray analysis including labelling, hybridization, scanning, normalization and data analysis is commercially available from a number of sources, for example, from Exiqon A/S. Briefly, RNA Quality Control is performed using Bioanalyser 2100 microfluidics platform (Bioanalyser is a trade mark of Agilent Technologies). Samples are labelled using the Complete Labelling Hyb Kit from Agilent, following the provided instructions.

### (2) Quantitative real-time PCR

As with option (1) above, all cellular RNA is extracted using a column-based kit from Exiqon and following the manufacturer's instructions. Quantification of miRNAs by TaqMan Real-Time PCR is carried out as described by the manufacturer (Applied Biosystems of Foster City, California, USA). (TaqMan is a trade mark of Roche Molecular Systems, Inc.). Briefly, 10 ng of RNA is used as a template for reverse transcription (RT) using the TaqMan MicroRNA Reverse Transcription Kit and miRNA-specific stem-loop primers (Applied Biosystems). An aliquot (1.5µl) of the RT product is introduced into 20 µl PCR reactions which are incubated in 96-well plates on the ABI 7900HT thermocycler (Applied Biosystems) at 95°C for 10 min, followed by 40 cycles of 95°C for 15 s and 60°C for 1 min. Target gene expression is normalized between different samples based on the values of U48 RNA (a small, non-coding RNA) expression (or U6 RNA, if U48 is found to vary between samples).

### Experimental Findings and their Implications.

Using the methods described we have established that it is possible to determine a novel way to monitor the identify the phenotypic drift of cells based on the grouping of miRNA expression data. Furthermore, the method can be employed to identify certain miRNAs, having expression levels which are indicative of potential alterations in cellular functions including pluripotentcy and tumourigenicity. These miRNAs will enable future intervention screening to analyse a relatively small group of miRNA expression levels changes to identify key alterations in cell physiology/pathophysiology with specific subsets, and not the entire miRNA repertoire, being used depending on the particular endpoint being investigated.

An example of using a select small group of miRNAs to determine potential Safety of a human embryonic stem cell population is given below.

### Materials and Methods

### RCM1 Cell Culture.

### Derivation

The cell line RCM-1 was derived from a freshly received Day 6 Blastocyst. It was manually hatched using a Swemed Stem Cell cutting tool (Vitrolife AB, Cat No: 14601) and the inner cell mass isolated and plated onto human fibroblasts (Cascade Biologics). The fibroblasts had been pre-plated onto tissue culture wells which in turn had been pre-coated with a layer of human Laminin (Sigma, Cat No: L4544). The cells were cultured in conditioned medium containing 24ng/ml human basic fibroblast growth factor (hbFGF) (Invitrogen, Cat No: PHG0261). The resultant outgrowth was manually passaged using a Swemed Stem Cell cutting tool and through early expansion continued to display a typical undifferentiated morphology while on the laminin/feeders plus hbFGF culture system.

The characteristics of the cell line represented in the summary document available online at http://www.roslincells.com/sitepix/downloads/RCM-1.pdf

### Expansion

RCM-1 was then adapted to a feeder-free culture system of CellSTART matrix (CS) (Invitrogen, Cat No: A10142-01) with StemPRO (SP) (Invitrogen, Cat No: A1000701) medium containing 8ng/ml hbFGF and under these conditions has maintained an undifferentiated morphology. The cell line was expanded through a number of passages using mechanical/manual methods in preference to enzymatic methods. At various passage stages, during the expansion of the cell line, cells were cryopreserved, as described and following manufactures instructions, using CryoStor CS10 (Stemcell Technologies, Cat No: 07930).

### Recovery from Cryopreservation

Three passage time-points, early, mid and late were thawed for the study, namely passages P38, P51 and P103.

Vials, in triplicate, were removed from -150°C freezer and quickly thawed at 37°C. The thawed cells were them washed twice in pre-warmed medium before being resuspended in fresh pre-warmed medium and plated into wells in a culture system of CellSTART matrix (CS) (Invitrogen) with StemPRO (SP) media containing 8ng/ml hbFGF.
Cells were cultured for 7 days (Figure 1), with repeated medium changes, before harvesting for RNA extraction (see below).

### Flow Cytometry Analysis

The cells which were harvested for RNA extraction were also sampled to determine the expression of the multiple markers of pluripotency and differentiation.

A single cell suspension was made from the remaining cells in culture and stained for the various markers associated with either a differentiated or undifferentiated state. The markers stained for were: stage-specific embryonic antigen 1 (SSEA-1) where an up regulation is indicative of a differentiated state, stage-specific embryonic antigen 4 (SSEA-4) where an up regulation is indicative of an undifferentiated state and Oct3/4, a 34 kDa POU transcription factor that is expressed in embryonic stem (ES) cells and germ cells, and its expression is required to sustain cell self-renewal and pluripotency, using a Human and Mouse Pluripotent Stem Cell Analysis Kit (BD, Cat No: 560477).

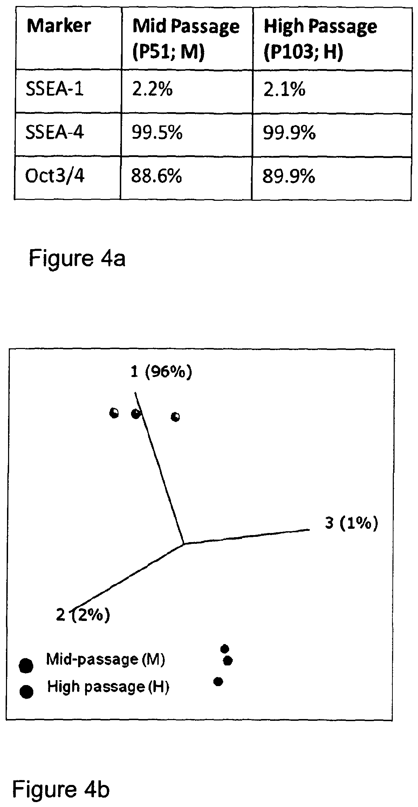

The stained cells are analysed using Flow Cytometry and the results produced give the status of the cell line both numerically and graphically for the markers analysed. Figure 4a.

### Tumour-derived Cell Lines

HeLa and MCF-7 cells were cultured and passaged (sub-cultured) using standard methods.

### RNA Extraction

Prior to miRNA profiling analysis, total RNA must be isolated from the cells, and analysed for quality. Total RNA from stem cells, at different passage numbers, is isolated using the miRCURY RNA isolation kit, obtainable from Exiqon (Denmark). Following the manufacturer's instructions, the cells are lysed in the tissue culture dish using a specific lysis buffer, and transferred to a column where the RNA is washed then eluted. RNA quantity and quality is checked using the Nanodrop ND-1000 spectrophotometer (Thermo Fisher of Waltham, MA, USA) and the Bioanalyser 2100 microfluids-based platform (Agilent Technologies of Santa Clara, CA, USA).

Micro RNA expression profiles for stem cell samples of different passage numbers can be determined by isolating total RNA from these samples and analysing them by two methods; (1) miRNA microarray and:

### (2) quantitative real-time PCR (QPCR).

Microarrays are used to achieve a complete miRNA profile of a sample, by collecting data on the expression levels of human 851 miRNAs simultaneously. QPCR is used to interrogate an individual miRNA of interest in a number of samples so differences in expression levels can be determined.

### (1) miRNA microarray and data analysis

Total RNA that has been checked for quality and has been diluted to an appropriate concentration is used as the starting material for miRNA profiling on the Agilent microarray platform. 100 ng of total RNA from each sample is processed through the microarray protocol, in which the microRNAs are labelled, hybridised to an array and scanned using the Agilent Microarray Scanner. Samples are labelled with Cy3 dye using the Agilent 'miRNA Complete Labeling and Hyb kit' and hybridised overnight on an Agilent miRNA array, 8 of which are found on each glass slide. On an array, each miRNA is represented 16 times, by at least 2 different probes. In addition, spike-in controls are used to evaluate the labelling and hybridisation efficiency of the reactions. Scanned images of the arrays constitute the input for the Agilent Feature Extraction software, which analyses each spot on the image, assigning it to a specific miRNA and calculating a value for the emitted fluorescent signal. The output from this processing is a series of QC reports, which evaluate the quality of the array processing, and text files, which contain the raw microarray data. These text files form the basis of the statistical analysis which is used to identify changes in miRNA expression between different samples. For best experimental design, biological replicates (n=3) are processed on different slides to ensure reproducibility. Microarray data is interpreted by statistical analysis programs such as GeneSpring (Agilent Technologies) and/or Omics Explorer (Qlucore of Lund, Sweden), and by Sistemic's in-house statistical methods (see below).

### RNA extraction

RNA was isolated and purified from these cells using a column-based kit from Exiqon the following procedure. The medium the cells were grown on was aspirated and the cell monolayer was washed with an appropriate amount of PBS. The PBS was further aspirated. 350 µL of the lysis solution was added directly to a culture plate. The cells were lysed by gently tapping the culture dish and swirling buffer around the plate surface for five minutes. The lysate was then transferred to a micro-centrifuge tube. 200 µL of 95-100% ethanol was added to the lysate and mixed by vortexing for 10 seconds. A column was assembled using one of the tubes provided 1 in the kit. 600 µL of the lysate/ethanol was applied onto the column and centrifuged for 1 minute at 14,000 xg. The flow-through was discarded and the spin column was reassembled with its collection tube. 400 µL of the supplied wash solution was applied to the column and centrifuged for 1 minute at 14,000 x g. The flow-through was discarded and the spin column was reassembled with its collection tube. The column was washed twice more by adding another 400 µL of wash solution and centrifuging for 1 minute at 14,000 x g. The flow-through was discarded and the spin column was reassembled with its collection tube. The column was spun for two minutes at 14,000 x g to thoroughly dry the resin and the collection tube was discarded. The column was assembled into a 1.7 mL elution tube provided with kit. 50 µL of elution buffer was added to the column and centrifuged for two minutes at 200 x g followed by one minute at 14,000 x g. The resulting purified RNA sample could be stored at -20°C for a few days. For long22 term storage of samples were stored at -70°C.

### (1) miRNA microarray and data analysis

### Labelling

Purified RNA samples were labelled using a labelling kit from Agilent. The total RNA sample was diluted to 50 ng/µL in 1 x TE pH 7.5. 2 µL of the diluted total RNA was added to a 1.5 mL micro-centrifuge tube and put on ice. Immediately prior to use, 0.4 µL 10 x calf intestinal phosphatase buffer, 1.1 µL nuclease free water and 0.5 µL calf intestinal phosphatase were gently mixed to prepare a calf intestinal alkaline phosphatase master mix. 2 µL of the calf intestinal alkaline phosphatase master mix was added to each sample tube for a total reaction volume 4 µL, and was gently mixed by pipetting. The reaction volume was incubated at 37°C in a circulating water bath for 30 minutes. 2.8 µL of 100% DMSO was added to each sample. Samples were incubated at 100°C in a circulating water bath for 5-10 minutes and then immediately transferred to an ice bath.

10 x T4 RNA ligase buffer was warmed to 37°C and spun until all precipitate had dissolved. Immediately prior to use, 1 µL of 10 x T4 RNA ligase buffer, 3 µL cyanine3-pCp and 0.5 µL T4 RNA ligase were gently mixed to make a ligation master mix and put on ice. 4.5 µL of the ligation master mix was added to each sample tube for a total reaction volume of 11.3 µL. Samples were gently mixed by pipetting and spun down. The samples were then incubated at 16°C in a circulating waterbath for two hours. The samples were then dried using a vacuum concentrator at 45-55°C and the samples were determined to be dry if, when the tube was flicked the pellets did not move or spread.

### Hybridization

125 µL of nuclease free water was added to the vial containing lyophilised 10 x GE blocking agent supplied with the Agilent Kit and mixed. The dried sample was resuspended in 18 µL of nuclease free water. 4.5 µL of the 10 x GE blocking agent was added to each sample. 22.5 µL of 2 x Hi- RPM Hybridization buffer was added to each sample and mixed well. The resulting samples were incubated at 100°C for 5 minutes, and then immediately transferred to an ice waterbath for a further 5 minutes. A clean gasket slide was loaded into the Agilent SureHyb chamber base ensuring the gasket slide was flush with the chamber base. The hybridization sample was dispensed onto the gasket well ensuring no bubbles were present.

An array was placed active side down onto the SureHyb gasket slide and assembled with the SureHyb chamber cover to form an assembled chamber. The assembled chamber was placed into 1 a hybridization oven set at 55°C and rotated at 20 rpm for 20 hours at that temperature.

The arrays were subsequently washed using the supplied GE wash buffers before being scanned.

### (2) Quantitative real-time PCR

Quantitative real-time PCR is carried out in three stages. The first two stages, to synthesise cDNA from the total RNA samples, use the qScript miRNA cDNA synthesis kit (Quanta Biosciences). The third step, QPCR reactions, use the SYBR Green PerfeCTa Low Rox Reaction Mix (Quanta Biosciences).

### Poly(A) Tailing Reaction

Total RNA samples (of between 100 ng and 1 µg) are aliquoted into fresh 0.5 ml tubes and made up to 7 µl with nuclease-free water. 2 µl of 5 x PAP (Poly(A) Polymerase) Tailing Buffer and 1 µl of Poly(A) Polymerase is added to each tube, then the tubes vortexed and centrifuged. The samples are then incubated in a thermal cycler under the following conditions: 37°C for 20 minutes, then 70°C for 5 minutes. Following this reaction, samples are placed on ice.

### cDNA Synthesis Reaction

A mastermix of RT is prepared so that each sample will receive 9 µl of miRNA cDNA Reaction Mix and 1 µl of qScript Reverse Transcriptase. 10 µl of this mix is added to each sample, then the tubes vortexed and centrifuged. The samples are then incubated in a thermal cycler under the following conditions: 42°C for 20 minutes, then 85°C for 5 minutes. Following this reaction, samples are placed on ice and then diluted 5-fold in 1 x TE buffer.

### QPCR Reaction

A mastermix of SYBR Green reaction mix and primers is prepared so that each sample well will receive the following kit components:
- 10 µl of 2 x SYBR Green PerfeCTa Low Rox Reaction Mix
- 0.4 µl of UA3PA Universal Reverse primer (10 □M)
- 0.4 µl of miRNA-specific primer (10 □M)
- 4.2 µl of nuclease-free water

To each well, 5 µl of cDNA is added. When all the wells are filled, the plate is sealed with plastic optical lids and centrifuged to remove air bubbles. The plate is loaded into the Agilent MX3005P thermocycler and processed under the following cycling conditions:
- 95°C for 2 minutes
- (95°C for 5 seconds, 60°C for 30 seconds) x 40 cycles
- Fluorescence data is collected at the end of every annealing/extension step

### Data Analysis

Data from both of these techniques was normalised against the spike-in miRNA spots for each plate, allowing data from separate arrays to be compared. Normalised data was analysed using Principal Component Analysis, a standard technique well understood by those skilled in the art to identify correlations between miRNA expression profiles, and any grouping of data observed determined to be a 15 consequence of the action of the particular test condition in relation to the original cells on the expression of the individual miRNA.

Figure 1 is a flow diagram of a method for obtaining an expression profile for micro RNA.

Figure 2 shows the alterations in has-miR-210, hsa-miR1274a and hsa-miR-302c* between passage numbers identified by microarray analysis and confirmed by QPCR measurements of the mature microRNAs.

Figure 3 shows alterations in microRNA profiles in a serially-passaged human, tumour-derived cell lines (HeLa and MCF-7).
As can be seen in Figure 2, the results are clearly grouped and that this grouping is according to the passage number of the cells in which the miRNAs were expressed. In other words, it is possible to determine that the replicate samples of identically-passaged cells have similar but distinct miRNA expression profiles.

A database of miRNA expression patterns can be built up by carrying out many comparisons of cell passage number and analysing the resulting changes in miRNA expression. Such a database would enable identification of phenotypic drift in pluripotent stem cells, or cell lines used in bioprocessing and indicate a loss of optimal functionality, in the former case pluripotent potential, in the latter case productions of a desired macromolecule. Furthermore, building up a database of miRNA expression data may reveal a subset of certain miRNAs that are indicative of an unfavourable or undefined alterations to cell physiology. Once subsets of indicative miRNAs are identified, future testing of new cell lines can be carried out by looking at the expression profiles of the subset of indicative miRNA expression profiles and not the entire range of miRNAs produced by the cells. miRNAs may be ranked in order of the relevance of their expression levels for discriminating between biological interventions, or between groups of interventions known or hypothesized to have similar effects on cell physiology. miRNAs may be allocated a numerical value indicative of the relevance of their expression levels for discriminating between interventions, or between groups of interventions known or hypothesized to have similar effects on the cells. For example, the numerical value may be related to the contribution of the expression level of a miRNA to the variance of principle components. As an alternative to, or in addition to, the comparison of miRNA expression profiles using statistical methods such as principal component analysis, the effect cell culture passages on the expression of each of a limited group of miRNAs (for example, 10-50) may be identified and used to assign a code, selected from a group of codes, to the effect of the biological intervention on the expression of each respective miRNA. The resulting codes may be compared to identify similarities in effect.

For example, for comparison (e.g. cell passage number) a 3-digit binary number may be allocated as a code to each ranked miRNA based on:
1. If expression of the miRNA is unchanged (within normal limits of experimental variability) in response to the biological intervention, the first bit is set to 0. If expression has changed significantly, the first bit is set to 1.
2. If a change in expression level was identified and the change was an increase, the second bit is set to 1. If the change resulting from the biological intervention was a decrease, the second bit is set to 0.
3. If the change in expression level was more than 4-fold, the third bit is set to 1, otherwise it is set to 0.

Thus, the effect of a difference between cell passages or culture conditions on the expression of a miRNA is allocated a code having one of five possible values:
1. No change 2 in expression - 000
2. Large increase in expression - 111
3. Small increase in expression - 110
4. Large decrease in expression - 101
5. Small decrease in expression - 100

The effect extended time in culture (i.e., an increase in passage number) on the expression level of a group of miRNAs may be characterised by the associated code, permitting identification of changes in expression level not immediately apparent from principal component analysis, permitting alternative methods of scoring the similarity of test conditions or interventions and rendering the resulting expression data comprehensible by visual inspection.

Another way to characterise the effect of a cell maintenance regime and to determine correlations between the effects on miRNA expression of different biological interventions is to carry out an expression assay to determine the effects of an intervention on the expression of each of a group (of typically 10 to 50) miRNAs and to rank the miRNAs in that group in order of the effect, for example, in order from the miRNA in the group which has the largest increase in expression to the miRNA in the group which has the largest decrease in expression, or vice versa. The resulting rankings are indicative of the effects of particular test point or interventions. Thus, the effect of other interventions on the group of miRNAs may be measured and the miRNAs in the group ranked in order of the effect. The resulting rankings may be compared to enable correlations between the effects of interventions to be identified.

A kit comprising plates operable to test the subset of indicative miRNAs may be provided to significantly increase the efficiency and speed with which the effect of cell passage and/or interventions can be screened for potential novel therapeutic applications.

### References

1. Xie, X., et al., Systematic discovery of regulatory motifs in human promoters and 3'-UTRs by comparison of several mammals. Nature, 2005. 434(7031): p. 338-45
2. Lim, L.P., et al., Microarray analysis shows that some microRNAs downregulate large numbers of target mRNAs. Nature, 2005. 433(7072): p.769-73
3. Calin, G.A., et al., MicroRNA profiling reveals distinct signatures in B cell chronic lymphocytic leukemias. Proc Natl Acad Aci USA, 2004. 101 (32): p. 11755-60

### EXAMPLE 2

### Summary

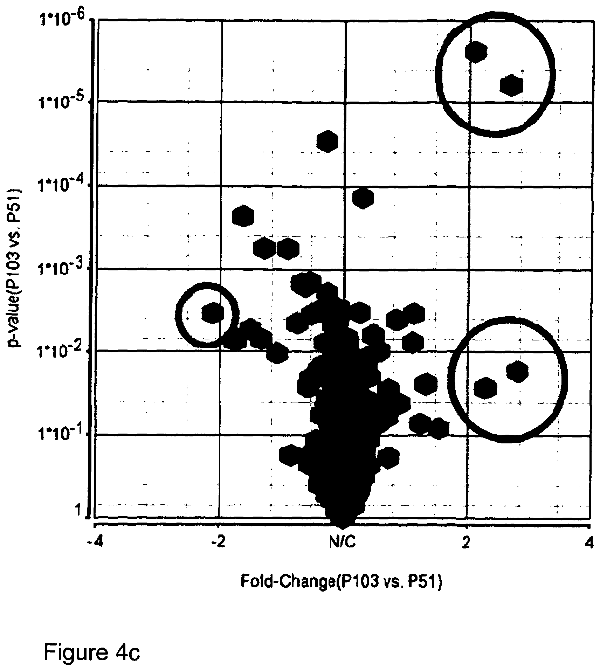

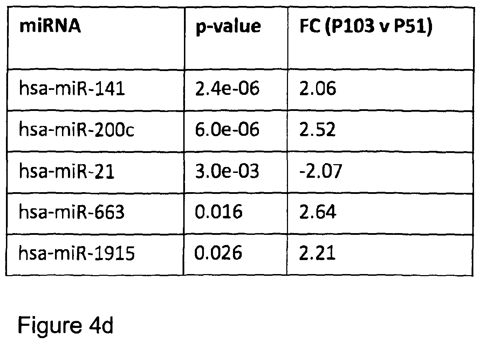

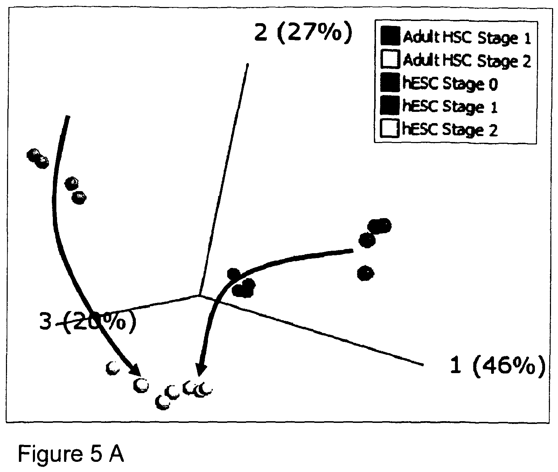

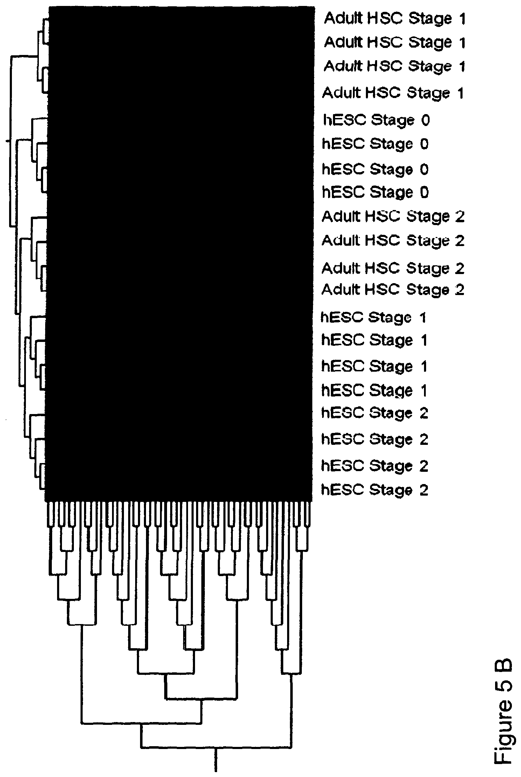

1. MicroRNA profiling of serially-passaged stem cells reveals differences in cells assessed to be 'identical' populations using flow cytometry and a commercial kit assessing cell surface and internal protein antigen markers of pluripotency and differentiation. (Figure 4)
2. micro-RNAs can be used to monitor the directed differentiation of hESC to erythrocytes by comparing miRNA profiles from two populations of CD34+ cells derived by directed differentiation of human embryonic cell lines (hESCs) for comparison with the equivalent developmental stage of adult CD34+ haematopoietic stems cells (HSCs; Figure 5).

### Methods.

These are outlined in Example 1 above (see section headed "Flow Cytometry" and "Data analysis" - in particular, PCA).

The hierarchical clustering and heatmap visualisation of the data were achieved using Qlucore Omics Explorer (Qlucore AB).

A volcano plot is a graphical representation of that is used to quickly identify changes in large datasets composed of replicate data. It plots significance versus fold-change on the y- and x-axes, respectively. The volcano plot was generated using the results of an ANOVA analysis for the hESC datasets. Both the ANOVA analysis and Volcanoes plot were generated using Partek's Genomic Suite (Partek, Inc).

### Results and discussion.

### 1. Identification of miRNA differences in pluripotent hESC cell populations otherwise assessed to be identical.

Roslin Cellabs utilised a human embryonic stem cell line, RCM1. Cells were obtained at mid-passage 51 (P51) and a late passage (P103), where an individual passage (i.e. the period between cell sub-culturing) is about 1-week. The cells were grown for up to three passages post-resuscitation from liquid nitrogen storage in order to generate sufficient cells for analysis by flow cytometry and miRNA profiling.

### Flow cytometry, MicroRNA profiling and data analysis

The cells from each passage were analysed using Flow Cytometry carried out by Roslin Cells. This analysis suggests that both cell populations are indistinguishable for the pluripotentcy and differentiation markers used in the commercial test (Figure 4a). However, as can be seen in Figure 4b below, the biological replicates (n=3) at each passage clearly group together according to the passage number of the cells in which the miRNAs were expressed. In other words, it is possible to determine that the replicate samples of identically-passaged cells have similar but distinct miRNA expression profiles.

There were 5 differentially-expressed miRNAs with a fold-change difference of 2 or more Figure 4c and the identity of these miRNAs are given in Figure 4d.

### 2. Monitoring hESC-derived and adult haematopoietic stem cells directed to differentiate to erythrocytes.

A PCA of the top 50 most variable miRNA transcripts is shown in Figure 5A below. The samples cluster distinctly based on cell type and stage, which is also evident from the heatmap in Figure 5B. For stage 1, the hESC and Adult HSC categories occupy separate spaces on the PCA plot, implying that these cell types have distinctly different properties. At stage 2, however, the hESC and Adult HSC categories are largely grouping together, demonstrating that the miRNA profiles of the samples are highly similar.

The following sample groups were analysed:
∘ hESC stage 0: Undifferentiated hESC
∘ hESC stage 1: hESC at day 10 of the differentiation protocol
∘ hESC stage 2: hESC at day 24 of the differentiation protocol
∘ Adult HSC stage 1: Adult HSC cells at a differentiation stage equivalent to that of hESC stage 1
∘ Adult HSC stage 2: Adult HSC cells at a differentiation stage equivalent to that of hESC stage 2 (14 days after induction of differentiation)

Further disclosed is: A method comprising steps of:
i. Growing cell lines as serially passaged cultures and at each passage where the cells are sampled, determining the microRNA expression profile, for example by microarraying, following a defined intervention or where there is no change to the growth conditions
ii. Define using a appropriate statistical test, for example Principal Components Analysis, separation between samples based on passage number, alterations to growth conditions, treatment with drugs or other external factors, transfection/viral transduction of gene(s)
iii. Determining the microRNAs which define the variation between the test conditions These miRNAs can inform the 'drift' of the cells from optimally pluripotent, optimally differentiating and/or optimally growing cells population and/or those safe for their purpose in bioprocessing, drug discovery or regenerative medicine i.e. reveal key information on the identity, purity, potency or safety (tumourigenicity of stem cells, microbiological contamination) of the cell population

Where the cells are mammalian (possibly human and/or rodent) undifferentiated, pluripotent, embryonic stem cells or iPS cells (where iPS cells (induced pluripotent stem cells) are defined as adult somatic cells which have been reprogrammed by direct expression of exogenous cDNAs/mRNAs/miRs from one or more transduced vectors). In combinations that may include chemical entities necessary for their production.

Where the cells are a mixture of one or more of the primary germ layers or progenitor cells derived from hESC or iPS cells

Where the cells are mirPS cells (from Mello Inc) or other cells reprogrammed by direct expression of exogenous miRNA(s) form one or more transduced vectors.

Where the biological system represents plasmid-based assay systems, controllably inserted into the hESC genome and have them actively express in pluripotent as well as in differentiated lineages derived from the genetically engineered cells.

Where tissue-specific stem cells are used to produce one or more terminally differentiated lineages following exposure to biological factors and/or chemical entities that direct differentiation

Where the cells are human or animal multipotent mesenchymal stem cells or any other adult stem cell population

Where the cells are primary cell cultures derived from human or animal tissues

Where the cells are established cell lines with and without genetic modifications (e.g. with virus or plasmid-based expression of an exogenous enzyme, protein or peptide)

Where the change in growth conditions includes alterations in cell matrix, including switching from 2-dimensional to 3-dimensional culture systems, cell media composition, addition of xenogenic components, drugs, excipients and chemicals, including those used for cosmetics, exposure to biological agents & their biosimilars, variations physical conditions (e.g temperature, radiation etc.).

Monitor commitment towards specific lineages following exposure to small molecules and biological factors (biologics or biosimilars), either alone or in combination.

For bioprocessing application specifically, monitor the effects in alterations dues to pH, osmolarity etc.

Others relating to the way the microRNAs are changing - positive or negative correlations as well as combinations of microRNA changes i.e. the pattern of miR changes defines the alteration to cell phenotype.

## Claims

1. A method of quality assessing stem cells or induced pluripotent stem cells or their intermediate stages of differentiating to one or more terminal differentiating states for use in cell therapy for levels of cell contamination, said method comprising the steps of
(i) determining a micro-RNA expression profile of the stem cells or induced pluripotent stem cells or their intermediate stages of differentiating to one or more terminal differentiating states to be assessed for a particular group or subset of micro-RNAs;
(ii) providing a reference micro-RNA expression profile of the particular group or subset of micro-RNAs which is derived from a cell sample of stem cells or induced pluripotent stem cells or their intermediate stages of differentiating to one or more terminal differentiating states that conforms to a predetermined standard for the use of stem cells or induced pluripotent stem cells or their intermediate stages of differentiating to one or more terminal differentiating states in the cell therapy;
(iii) comparing the micro-RNA expression profile determined in step (i) with the reference micro-RNA expression profile provided in step (ii); and
(iv) determining from the comparison of micro-RNA expression profiles a quality assessment as to the suitability of the stem cells or induced pluripotent stem cells or their intermediate stages of differentiating to one or more terminal differentiating states for use in the cell therapy.

2. The method as claimed in claim 1, wherein the qualities being assessed further comprise the maintenance of cell identity, phenotypic stability and functional capability and wherein the particular group or subset of micro-RNAs includes micro-RNAs derived from cells of known identity and/or known phenotypic stability and/or known functional capability and determined to be a marker of the cells identity, phenotypic stability or functional capability.

3. The method as claimed in claim 1 or claim 2, wherein conformity to pre-determined safety standards of the stem cells for use in stem cell therapy is thereby assessed.

4. The method as claimed in claim 3, wherein predetermined safety standard concerns tumourigenicity.

5. The method as claimed in any one of the preceding claims, wherein the particular group or subset of micro-RNAs includes micro-RNAs that are identified as reliable markers of undesirable or uncharacterized alterations of the cell system being monitored for stem cell therapy.

6. The method as claimed in any one of the preceding claims, wherein the cells to be assessed are stem cells.

7. The method as claimed in any one of claims 1 to 6, wherein the stem cells to be quality assessed for use in stem cell therapy are cultured from an in vitro cell culture and/or passage thereof.

8. The method as claimed in any one of claims 1 to 7, wherein the stem cells are mesenchymal stem cells.

9. The method as claimed in any one claims 1 to 5, wherein the cells to be assessed are induced pluripotent stem cells or their intermediate stages of differentiating to one or more terminal differentiating states.

10. The method as claimed in claim 9, wherein the quality assessing further comprises assessing degree of pluripotency.

11. The method as claimed in any one of the preceding claims, wherein the step of comparing the micro-RNA expression profile of the cell sample with the reference expression profile comprises identifying correlations between micro-RNA profiles.

12. A method as claimed in claim 11, wherein the correlations may comprise positive and/or negative correlations between the expression of one or more micro-RNAs.

13. A method as claimed in claim 12, wherein a positive correlation is an expression of a micro-RNA in the sample to be quality assessed and in the reference micro-RNA profile and a negative correlation is expression of a micro-RNA in the sample to be quality assessed which exhibits differential expression in the reference microRNA profile.

14. A method as claimed in any one of the preceding claims, wherein the method is other than to distinguish one differentiative cell state from another.

15. Use of micro-RNA molecules for quality assessing stem cells or induced pluripotent stem cells or their intermediate stages of differentiating to one or more terminal differentiating states for use in cell therapy, wherein the qualities being assessed comprise levels of cell contamination.

## Patentansprüche

1. Verfahren zur Qualitätsbewertung von Stammzellen oder induzierten pluripotenten Stammzellen oder ihren Zwischenstufen der Differenzierung in einen oder mehrere terminale Differenzierungszustände zur Verwendung in der Zelltherapie für Zellkontaminierungsgrade, wobei das Verfahren die Schritte umfasst
(i) Bestimmen eines microRNA-Expressionsprofils der Stammzellen oder induzierter pluripotenter Stammzellen oder ihrer Zwischenstufen der Differenzierung in einen oder mehrere terminale Differenzierungszustände zur Bewertung einer bestimmten Gruppe oder Teilgruppe der microRNAs;
(ii) Bereitstellen eines Referenz-microRNA-Expressionsprofils der bestimmten Gruppe oder Teilgruppe von microRNAs, das aus einer Zellprobe von Stammzellen oder induzierten pluripotenten Stammzellen oder ihren Zwischenstufen der Differenzierung in einen oder mehrere terminale Differenzierungszustände stammt, das mit einem vorgegebenen Standard für die Verwendung von Stammzellen oder induzierten pluripotenten Stammzellen oder ihren Zwischenstufen der Differenzierung in einen oder mehrere terminale Differenzierungszustände bei der Zelltherapie übereinstimmt;
(iii) Vergleichen des microRNA-Expressionsprofils, das in Schritt (i) bestimmt wurde, mit dem Referenz-microRNA-Expressionsprofil, das in Schritt (ii) bereitgestellt wurde; und
(iv) Bestimmen einer Qualitätsbewertung aus dem Vergleich von microRNA-Expressionsprofilen bezüglich der Eignung der Stammzellen oder induzierter pluripotenter Stammzellen oder ihrer Zwischenstufen der Differenzierung in einen oder mehrere terminale Differenzierungszustände zur Verwendung in der Zelltherapie.

2. Das Verfahren nach Anspruch 1, wobei die Qualitäten, die bewertet werden, weiter die Aufrechterhaltung von Zellidentität, phänotypischer Stabilität und Funktionsfähigkeit umfassen und wobei die bestimmte Gruppe oder Teilgruppe der microRNAs microRNAs einschließt, die von Zellen mit bekannter Identität und/oder bekannter phänotypischer Stabilität und/oder bekannter Funktionsfähigkeit stammen und als Marker der Zellidentität, phänotypischer Stabilität oder Funktionsfähigkeit bestimmt werden.

3. Das Verfahren nach Anspruch 1 oder Anspruch 2, wobei die Konformität mit den vorgegebenen Sicherheitsstandards der Stammzellen zur Verwendung in der Stammzellentherapie bewertet wird.

4. Das Verfahren nach Anspruch 3, wobei der vorgegebene Sicherheitsstandard Tumorigenität betrifft.

5. Das Verfahren nach einem der vorstehenden Ansprüche, wobei die bestimmte Gruppe oder Teilgruppe der microRNAs microRNAs einschließt, die als zuverlässige Marker unerwünschter oder nicht charakterisierter Änderungen des zur Stammzellentherapie beobachteten Zellsystems identifiziert werden.

6. Das Verfahren nach einem der vorstehenden Ansprüche, wobei die zu bewertenden Zellen Stammzellen sind.

7. Das Verfahren nach einem der Ansprüche 1 bis 6, wobei die Stammzellen, die zur Verwendung bei der Stammzellentherapie qualitätsbewertet werden, aus einer in vitro Zellkultur und/oder einer Passage davon kultiviert werden.

8. Das Verfahren nach einem der Ansprüche 1 bis 7, wobei die Stammzellen mesenchymale Stammzellen sind.

9. Das Verfahren nach einem der Ansprüche 1 bis 5, wobei die zu bewertenden Zellen induzierte pluripotente Stammzellen oder ihre Zwischenstufen der Differenzierung in einen oder mehrere terminale Differenzierungszustände sind.

10. Das Verfahren nach Anspruch 9, wobei die Qualitätsbewertung weiter die Bewertung des Pluripotenzgrades umfasst.

11. Das Verfahren nach einem der vorstehenden Ansprüche, wobei der Schritt des Vergleichens des microRNA-Expressionsprofils der Zellprobe mit dem Referenzexpressionsprofil das Identifizieren von Korrelationen zwischen microRNA-Profilen umfasst.

12. Verfahren nach Anspruch 11, wobei die Korrelationen positive und/oder negative Korrelationen zwischen der Expression einer oder mehrerer microRNAs umfassen können.

13. Verfahren nach Anspruch 12, wobei eine positive Korrelation eine Expression einer microRNA in der Probe, die qualitätsbewertet werden soll, und in dem Referenz-microRNA-Profil ist, und eine negative Korrelation die Expression einer microRNA in der Probe, die qualitätsbewertet werden soll, ist, welche differentielle Expression in dem Referenz-microRNA-Profil zeigt.

14. Verfahren nach einem der vorstehenden Ansprüche, wobei das Verfahren etwas anderes ist als einen differentiativen Zellzustand von einem anderen zu unterscheiden.

15. Verwendung von microRNA-Molekülen zur Qualitätsbewertung von Stammzellen oder induzierten pluripotenten Stammzellen oder ihren Zwischenstufen der Differenzierung in einen oder mehrere terminale Differenzierungszustände zur Verwendung in der Zelltherapie, wobei die Qualitäten, die bewertet werden, Kontaminierungsgrade umfassen.

## Revendications

1. Procédé d'évaluation de la qualité de cellules souches ou de cellules souches pluripotentes induites ou de leurs stades intermédiaires de différenciation vers un ou plusieurs états terminaux de différenciation pour une utilisation dans une thérapie cellulaire pour des niveaux de contamination cellulaire, ledit procédé comprenant les étapes de :
(i) détermination d'un profil d'expression de micro-ARN des cellules souches ou des cellules souches pluripotentes induites ou de leurs stades intermédiaires de différenciation vers un ou plusieurs états terminaux de différenciation à évaluer pour un groupe ou sous-ensemble particulier de micro-ARN ;
(ii) fourniture d'un profil d'expression de micro-ARN de référence du groupe ou sous-ensemble particulier de micro-ARN qui est dérivé d'un échantillon cellulaire de cellules souches ou de cellules souches pluripotentes induites ou de leurs stades intermédiaires de différenciation vers un ou plusieurs états terminaux de différenciation qui est conforme à une norme prédéterminée pour l'utilisation de cellules souches ou de cellules souches pluripotentes induites ou de leurs stades intermédiaires de différenciation vers un ou plusieurs états terminaux de différenciation dans la thérapie cellulaire ;
(iii) comparaison du profil d'expression de micro-ARN déterminé dans l'étape (i) au profil d'expression de micro-ARN de référence fourni dans l'étape (ii) ; et
(iv) détermination à partir de la comparaison des profils d'expression de micro-ARN d'une évaluation de qualité quant au caractère approprié des cellules souches ou des cellules souches pluripotentes induites ou de leurs stades intermédiaires de différenciation vers un ou plusieurs états terminaux de différenciation pour une utilisation dans la thérapie cellulaire.

2. Procédé selon la revendication 1, dans lequel les qualités évaluées comprennent en outre la conservation d'une identité cellulaire, d'une stabilité phénotypique et d'une capacité fonctionnelle et dans lequel le groupe ou sous-ensemble particulier de micro-ARN inclut des micro-ARN dérivés de cellules d'identité connue et/ou de stabilité phénotypique connue et/ou de capacité fonctionnelle connue et déterminés comme étant un marqueur de l'identité, de la stabilité phénotypique ou de la capacité fonctionnelle des cellules.

3. Procédé selon la revendication 1 ou la revendication 2, dans lequel la conformité à des normes de sécurité prédéterminées des cellules souches pour une utilisation dans une thérapie avec des cellules souches est ainsi évaluée.

4. Procédé selon la revendication 3, dans lequel la norme de sécurité prédéterminée concerne la tumorigénicité.

5. Procédé selon l'une quelconque des revendications précédentes, dans lequel le groupe ou sous-ensemble particulier de micro-ARN inclut des micro-ARN qui sont identifiés en tant que marqueurs fiables d'altérations indésirables ou non caractérisées du système cellulaire surveillé pour une thérapie avec des cellules souches.

6. Procédé selon l'une quelconque des revendications précédentes, dans lequel les cellules à évaluer sont des cellules souches.

7. Procédé selon l'une quelconque des revendications 1 à 6, dans lequel les cellules souches dont la qualité doit être évaluée pour une utilisation dans une thérapie avec des cellules souches sont cultivées à partir d'une culture de cellules in vitro et/ou d'un passage de celle-ci.

8. Procédé selon l'une quelconque des revendications 1 à 7, dans lequel les cellules souches sont des cellules souches du mésenchyme.

9. Procédé selon l'une quelconque des revendications 1 à 5, dans lequel les cellules à évaluer sont des cellules souches pluripotentes induites ou leurs stades intermédiaires de différenciation vers un ou plusieurs états terminaux de différenciation.

10. Procédé selon la revendication 9, dans lequel l'évaluation de la qualité comprend en outre l'évaluation du degré de pluripotence.

11. Procédé selon l'une quelconque des revendications précédentes, dans lequel l'étape de comparaison du profil d'expression de micro-ARN de l'échantillon cellulaire au profil d'expression de référence comprend l'identification de corrélations entre les profils de micro-ARN.

12. Procédé selon la revendication 11, dans lequel les corrélations peuvent comprendre des corrélations positives et/ou négatives entre l'expression d'un ou de plusieurs micro-ARN.

13. Procédé selon la revendication 12, dans lequel une corrélation positive est une expression d'un micro-ARN dans l'échantillon dont la qualité doit être évaluée et dans le profil de micro-ARN de référence et une corrélation négative est l'expression d'un micro-ARN dans l'échantillon dont la qualité doit être évaluée qui présente une expression différentielle dans le profil de micro-ARN de référence.

14. Procédé selon l'une quelconque des revendications précédentes, dans lequel le procédé est autre que le fait de distinguer un état cellulaire de différenciation d'un autre.

15. Utilisation de molécules de micro-ARN pour évaluer la qualité de cellules souches ou de cellules souches pluripotentes induites ou de leurs stades intermédiaires de différenciation vers un ou plusieurs états terminaux de différenciation pour une utilisation dans une thérapie cellulaire, dans laquelle les qualités évaluées comprennent des niveaux de contamination cellulaire.
